(19) Europäisches Patentamt
European Patent Office
Office européen des brevets

(11) **EP 2 803 730 A1**

(12) **EUROPEAN PATENT APPLICATION**
published in accordance with Art. 153(4) EPC

(43) Date of publication:
**19.11.2014 Bulletin 2014/47**

(51) Int Cl.:
***C12P 7/06*** (2006.01)       ***C12P 7/56*** (2006.01)

(21) Application number: **13735950.1**

(86) International application number:
**PCT/JP2013/050437**

(22) Date of filing: **11.01.2013**

(87) International publication number:
**WO 2013/105652 (18.07.2013 Gazette 2013/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.01.2012   JP 2012005256**

(71) Applicant: **Toray Industries, Inc.**
**Tokyo, 103-8666 (JP)**

(72) Inventors:
• **WATANABE, Shiomi**
  **Kamakura-shi**
  **Kanagawa 248-8555 (JP)**
• **KOBAYASHI, Koji**
  **Kamakura-shi**
  **Kanagawa 248-8555 (JP)**

• **ISOBE, Kyohei**
  **Kamakura-shi**
  **Kanagawa 248-8555 (JP)**
• **SAWAI, Kenji**
  **Kamakura-shi**
  **Kanagawa 248-8555 (JP)**
• **NA, Kyungsu**
  **Kamakura-shi**
  **Kanagawa 248-8555 (JP)**
• **HIRAMATSU, Shingo**
  **Kamakura-shi**
  **Kanagawa 248-8555 (JP)**
• **YAMADA, Katsushige**
  **Kamakura-shi**
  **Kanagawa 248-8555 (JP)**

(74) Representative: **Kador & Partner**
**Corneliusstraße 15**
**80469 München (DE)**

(54) **METHOD FOR PRODUCING CHEMICAL SUBSTANCE**

(57)     [PROBLEMS] To provide a method for producing a chemical product with a high yield using a mixed sugar of hexose and pentose as a fermentation feedstock. [MEANS FOR SOLUTION] A method for producing a chemical product by continuous fermentation, which method comprises filtering a culture liquid of a microorganism(s) through a separation membrane; retaining unfiltered liquid in, or refluxing unfiltered liquid to, the culture liquid; adding a fermentation feedstock to the culture liquid; and recovering a product in the filtrate, wherein the fermentation feedstock contains pentose and hexose, and wherein the microorganism(s) is/are a microorganism(s) having a pathway in which pentose reductase and pentol dehydrogenase are used to metabolize pentose, was provided.

**EP 2 803 730 A1**

**Description**

TECHNICAL FIELD

The present invention relates to a method for producing a chemical product by continuous fermentation using a fermentation feedstock containing hexose and pentose.

BACKGROUND ART

As the problem of carbon dioxide emission into the atmosphere and the energy problem have been actualized, biomass-derived chemical products represented by biodegradable polymer materials such as lactic acid and biofuels such as ethanol have attracted stronger attention as products with sustainability and life cycle assessment (LCA) capability. These biodegradable polymer materials and biofuels are generally produced as fermentation products from microorganisms using as a fermentation feedstock glucose, which is a hexose, purified from edible biomass such as maize. However, use of edible biomass may cause a rise in its price because of competition with food, resulting in unstable supply of the feedstock. In view of this, attempts are being made to use sugars derived from non-edible biomass such as rice straw as a fermentation feedstock for microorganisms (see Patent Document 1).

In cases where a sugar derived from non-edible biomass is used as a fermentation feedstock, cellulose, hemicellulose and the like contained in the non-edible biomass are decomposed into sugars by a saccharifying enzyme. In this process, not only hexoses such as glucose, but also pentoses such as xylose are obtained, and as a consequence a mixed sugar of hexose and pentose is used as a fermentation feedstock if a sugar derived from non-edible biomass is used as a fermentation feedstock for a microorganism (see Patent Document 1). In cases where a mixed sugar of hexose and pentose is used, a microorganism having not only a metabolic pathway for hexose but also a metabolic pathway for pentose needs to be used. As a metabolic pathway for pentose, a metabolic pathway using pentose reductase and pentol dehydrogenase is known, and it is known that, in this pathway, pentose reductase acts on hexose in the first step to produce pentol, and pentol dehydrogenase then acts on the pentol in the second step. However, the pentol produced in the first step does not flow into the pathway in the second and subsequent steps, and accumulates in the culture liquid, resulting in a low production yield, which is problematic.

As a fermentation method in which a sugar derived from non-edible biomass, which is a mixed sugar of hexose and pentose, is used as a fermentation feedstock for a microorganism, continuous fermentation may be employed, but the fermentation yield actually achieved by continuous fermentation has not been studied (see Patent Document 1). On the other hand, as known in the art, in the case where continuous fermentation is performed using a mixed sugar of hexose and pentose as a fermentation feedstock, the fermentation yield is lower than the case of batch fermentation (see Non-patent Document 1).

Thus, according to the common technical knowledge, in order to improve the fermentation yield in continuous fermentation in which a mixed sugar of hexose and pentose is used as a fermentation feedstock for a microorganism having a pentose metabolic pathway that uses pentose reductase and pentol dehydrogenase, it has been thought that improvement of the xylose metabolic pathway by introduction of a mutation or gene introduction is required.

PRIOR ART DOCUMENTS

PATENT DOCUMENT

Patent Document 1: WO2010/067785

NON-PATENT DOCUMENT

Non-patent Document 1: Susan T. Toon et al., Enhanced Cofermentation of Glucose and Xylose by Recombinant Saccharomyces Yeast Strains in Batch and Continuous Operating Modes., Applied Biochemistry and Biotechnology., (1997), 63-65, 243-255.

SUMMARY OF THE INVENTION

PROBLEMS TO BE SOLVED BY THE INVENTION

The present invention aims to increase the fermentation yield in fermentation using a mixed sugar of hexose and pentose as a fermentation feedstock for a microorganism having a pentose metabolic pathway that uses pentose reductase and pentol dehydrogenase.

MEANS FOR SOLVING THE PROBLEMS

As a result of intensive study of the problem, the present inventors solved the problem by performing continuous fermentation using a fermentation feedstock containing hexose and pentose with use of a separation membrane when a microorganism having pentose reductase and pentose dehydrogenase is used, to realize an increased yield from a mixed sugar of hexose and pentose, thereby reaching the present invention.

That is, the present invention is as described in (1) to (7) below.

(1) A method for producing a chemical product by continuous fermentation, said method comprising filtering a culture liquid of a microorganism(s) through a separation membrane; retaining unfiltered liquid in, or refluxing unfiltered liquid to, the culture liquid; adding a fermentation feedstock to the culture liquid; and recovering a product in the filtrate, wherein said fermentation feedstock contains pentose and hexose, and wherein said microorganism(s) is/are a microorganism(s) having a pathway in which pentose reductase and pentol dehydrogenase are used to metabolize pentose.

(2) The method for producing a chemical product according to (1), comprising performing continuous fermentation under conditions where the oxygen transfer coefficient (KLa) is 5 to 300 h$^{-1}$.

(3) The method for producing a chemical product according to (1) or (2), wherein the ratio between the pentose and hexose contained in said fermentation feedstock is 1:9 to 9:1.

(4) The method for producing a chemical product according to (1) or (2), wherein said fermentation feedstock comprises a biomass-derived sugar liquid.

(5) The method for producing a chemical product according to any one of (1) to (4), wherein said pentose is xylose.

(6) The method for producing a chemical product according to any one of (1) to (5), wherein said pentose reductase is xylose reductase.

(7) The method for producing a chemical product according to any one of (1) to (6), wherein said pentol dehydrogenase is xylitol dehydrogenase.

EFFECT OF THE INVENTION

By the present invention, the efficiencies and yields of fermentation production of various chemical products using a fermentation feedstock containing hexose and pentose by microorganisms having pentose reductase and pentose dehydrogenase can be largely increased.

BEST MODE FOR CARRYING OUT THE INVENTION

The method for producing a chemical product of the present invention is characterized in that the method is continuous fermentation in which a fermentation feedstock containing a mixed sugar of pentose and hexose is used to culture a microorganism(s) having pentose reductase and pentose dehydrogenase; the culture liquid is filtered through a separation membrane; the unfiltered liquid is retained in, or refluxed to, the culture liquid; the fermentation feedstock is added to the culture liquid; and a product in the filtrate is recovered.

Five-carbon sugar, also called pentose, has 5 carbons constituting the sugar. Pentose can be classified into aldopentose, which has an aldehyde group at the 1-position, and ketopentose, which has a ketone group at the 2-position. Examples of aldopentose include xylose, arabinose, ribose and lyxose, and examples of ketopentose include ribulose and xylulose. The pentose used in the present invention may be any pentose as long as it can be metabolized by a microorganism, and, in view of the abundance in nature, availability and the like, xylose and arabinose are preferred, and xylose is more preferred.

Six-carbon sugar, also called hexose, has 6 carbons constituting the sugar. Hexose can be classified into aldose, which has an aldehyde group at the 1-position, and ketose, which has a ketone group at the 2-position. Examples of aldose include glucose, mannose, galactose, allose, gulose and talose, and examples of ketose include fructose, psicose and sorbose. The hexose used in the present invention may be any hexose as long as it can be metabolized by a microorganism, and, in view of the abundance in nature, availability and the like, glucose, mannose and galactose are preferred, and glucose is more preferred.

The mixed sugar used in the present invention is not limited, and the mixed sugar is preferably a sugar liquid derived from a cellulose-containing biomass since it contains both hexose and pentose. Examples of the cellulose-containing biomass include herbaceous biomasses such as bagasse, switchgrass, corn stover, rice straw and wheat straw; and woody biomasses such as trees and waste building materials. Cellulose-containing biomasses contain cellulose or hemicellulose, which are polysaccharides produced by dehydration condensation of sugars. By hydrolyzing such polysaccharides, sugar liquids which may be used as fermentation feedstocks are produced. The method for preparing the sugar liquid derived from a cellulose-containing biomass may be any method, and examples of disclosed methods for producing such a sugar include a method in which a sugar liquid is produced by acid hydrolysis of a biomass using concentrated sulfuric acid (JP HI 1-506934 A, JP 2005-229821 A), and a method in which a biomass is subjected to hydrolysis treatment with dilute sulfuric acid and then enzymatically treated with cellulase and/or the like to produce a sugar liquid (A. Aden et al., "Lignocellulosic Biomass to Ethanol Process Design and Economics Utilizing Co-Current Dilute Acid Prehydrolysis and Enzymatic Hydrolysis for Corn Stover" NREL Technical Report (2002)). Further, examples of disclosed methods in which no acids are used include a method in which a biomass is hydrolyzed using subcritical water at about 250 to 500°C to produce a sugar liquid (JP 2003-212888 A), a method in which a biomass is subjected to subcritical water treatment and then enzymatically treated to produce a sugar liquid (JP 2001-95597 A), and a method in which a biomass is subjected to hydrolysis treatment with pressurized hot water at 240 to 280°C and then enzymatically treated to produce a sugar liquid (JP 3041380 B). These treatments may be followed by purification of the obtained sugar liquid. An example of the method is disclosed in WO2010/067785.

The weight ratio between the pentose and hexose contained in the mixed sugar may be any ratio, and preferably 1:9 to 9:1 as represented by the ratio of (pentose):(hexose) in terms of the weight ratio between pentose and hexose in the mixed sugar liquid. This is the sugar ratio for cases where the mixed sugar is assumed to be a sugar liquid derived from a cellulose-containing biomass.

The total sugar concentration in the mixed sugar is preferably high within the range in which the microorganism(s) does/do not undergo substrate inhibition. In cases where the sugar concentration is too low, the production efficiency is low, and therefore the sugar concentration is preferably not less than 20 g/L. A preferred range of the total sugar concentration is 20 to 500 g/L. In view of the above, the pentose concentration is preferably not less than 5 g/L, and the hexose concentration is preferably not less than 5 g/L.

Examples of the nitrogen source contained in the fermentation feedstock include ammonia gas, aqueous ammonia, ammonium salts, urea and nitric acid salts; and other organic nitrogen sources used supplementarily such as oilcakes, soybean-hydrolyzed liquids, casein digests, other amino acids, vitamins, corn steep liquors, yeasts or yeast extracts, meat extracts, peptides such as peptones, and cells of various fermentation microorganisms and hydrolysates thereof. Examples of inorganic salts that may be added as appropriate include phosphoric acid salts, magnesium salts, calcium salts, iron salts and manganese salts.

In cases where the microorganism(s) used in the present invention require(s) a specific nutrient for its/their growth, the nutrient is added as a preparation or as a natural product containing the nutrient. An anti-forming agent is added as required. In the present invention, the culture liquid means a liquid obtained as a result of growth of a microorganism(s) in a fermentation feedstock. The composition of the fermentation feedstock to be added may be changed as appropriate from the composition of the fermentation feedstock used at the beginning of the culture, such that the productivity of the chemical product of interest increases.

Next, microorganisms that may be used for the method for producing a chemical product of the present invention are explained below. The microorganism(s) in the present invention is/are not limited as long as the microorganism(s) has/have pentose reductase and pentose dehydrogenase. Further, the microorganism(s) used may be a microorganism(s) isolated from the natural environment, or a microorganism(s) whose properties are partially modified by mutation or genetic recombination.

Pentose reductase is a reducing enzyme, and means an enzyme having an activity to convert an aldopentose into a sugar alcohol using NADH or NADPH as a coenzyme. For example, EC 1.1.1.307 and EC 1.1.1.21 correspond to xylose reductase, which is an enzyme that converts xylose to xylitol. For example, EC 1.1.1.21 corresponds to arabinose reductase, which is an enzyme that converts arabinose to arabitol. Enzymes that are not categorized into the EC numbers described above are also included in the pentose reductase in the present invention as long as the enzymes have the activities described above.

Pentol dehydrogenase is a dehydrogenase, and means an enzyme that converts pentol to ketopentose using NAD+ as a coenzyme. For example, EC 1.1.1.9 and EC 1.1.1.10 correspond to xylose dehydrogenase, which is an enzyme that converts xylitol to xylulose. For example, EC 1.1.1.11 and EC 1.1.1.12 correspond to arabitol dehydrogenase, which is an enzyme that converts arabitol to ribose. Enzymes that are not categorized into pentol dehydrogenase are also included in the pentol dehydrogenase in the present invention as long as the enzymes have the activities described above.

Microorganisms that can metabolize pentose are known as microorganisms having pentose reductase and pentose dehydrogenase. Examples of such microorganisms include *Pichia, Candida, Pachysolen, Kluyveromyces, Hansenula, Torulopsis, Debaryomyces, Issachenkia, Brettanomyces, Lindnera* and *Wickerhamomyces*. Specific examples of such microorganisms include *Pichia stipitis, Pichia mexcana, Candida shehatae, Candida utilis, Candida tropicalis, Candida tenuis, Candida boidinii, Candida sonorensis, Candida diddensiae, Candida intermedia, Candida parapsilosis, Candida methanosorbosa, Candida wickerhamii, Pachysolen tannophilus, Kluyveromyces marxianus, Kluyveromyces lactis, Issachenkia orientalis, Debaryomyces hansenii, Hansenula polymorpha, Torulopsis bombicola, Brettanomyces naardenensis, Lindnera rhodanensis* and *Wickerhamomyces rabaulensis.* The fact that these have pentose reductase and pentose dehydrogenase can be easily known by searching databases published on the web such as those of KEGG (Kyoto Encyclopedia of Genes and Genomes) and GenBank.

Further, even in the case of microorganisms whose information is not available in databases, whether the microorganisms have pentose reductase and pentose dehydrogenase can be known by measuring the enzyme activities as described in [1] and [2] below.

[1] Measurement of Pentose Reductase Activity

The pentose reductase activity can be measured by adding a cell extract of a cultured microorganism to 50 mM phosphate buffer (900 μL) supplemented with 200 mM xylose (or pentose such as arabinose) and 100 μL of 1.5 mM NAD(P)H, and then monitoring at 30°C a decrease in the absorbance at 340 nm specific to NAD(P)+ produced by the reaction.

[2] Measurement of Pentol Dehydrogenase Activity

The pentol dehydrogenase activity can be measured by adding a cell extract of a cultured microorganism to 900 μL of 50 mM Tris-HCl buffer supplemented with 50 mM $MgCl_2$, 300 mM xylitol (or pentose such as arabinose) and 100 μL of 10 mM NAD(P)+, and then monitoring at 35°C an increase in the absorbance at 340 nm specific to NAD(P)H produced by the reaction.

Further, microorganisms such as bacteria and baker's yeasts that originally do not have the pentose reductase activity and the pentose dehydrogenase activity are also included in the present invention in cases where they are made to express the enzymes by genetic recombination. Known examples of microorganisms that originally do not have the pentose reductase activity and the pentose dehydrogenase activity include *Escherichia coli, Saccharomyces cerevisiae, Candida glabrata, Corynebacterium glutamicum, Bacillus coagulans, Bacillus subtillis, Sporolactobacillus laevolacticus, Paenibacillus polymixa, Zymomonas mobilis* and *Zymobacter palmae.* Examples of the method for preparing a microorganism that expresses the enzymes by genetic recombination include the method disclosed in JP 2009-112289 A.

Further, the microorganism having the enzymes may have fermentation capacity enhanced by introduction of a mutation or genetic recombination, or may have an exogenous gene introduced therein that makes the microorganism produce a substance that is not originally produced by the microorganism. More specifically, for example, xylose isomerase is introduced in addition to xylose reductase and xylitol dehydrogenase; high expression of xylulose kinase, which acts on xylulose, a metabolite from xylitol, is induced; or D-lactate dehydrogenase is introduced to a microorganism that does not produce D-lactic acid.

The porous membrane used as a separation membrane in the present invention is explained below.

The porous membrane used in the present invention is not limited as long as it has a function to separate a culture liquid obtained by culturing a microorganism(s) in a stirred culture vessel or a stirred bioreactor from the microorganism(s) by filtration. Examples of porous membranes that may be used include porous ceramic membranes, porous glass membranes, porous organic polymer membranes, metal fiber textiles, and non-woven fabrics. Among these, porous organic polymer membranes and ceramic membranes are especially preferred.

The constitution of the porous membrane used as the separation membrane in the present invention is explained below. The porous membrane used in the present invention has a separation performance and a permeability suitable for the properties and use of the liquid to be processed.

The porous membrane is preferably a porous membrane comprising a porous resin layer in view of the blocking performance, permeability and separation performance, for example, resistance to dirt.

The porous membrane comprising a porous resin layer preferably has the porous resin layer that functions as a separation functional layer on the surface of a porous base material. The porous base material supports the porous resin layer to give strength to the separation membrane.

In cases where the porous membrane used in the present invention has a porous resin layer on the surface of a porous base material, the porous base material may be impregnated with the porous resin layer or may not be impregnated with the porous resin layer, which may be selected depending on the use of the membrane.

The average thickness of the porous base material is preferably 50 μm to 3000 μm.

The porous base material is composed of an organic material and/or inorganic material etc., and an organic fiber is preferably used. Preferred examples of the porous base material include woven fabrics and non-woven fabrics composed of organic fibers such as cellulose fibers, cellulose triacetate fibers, polyester fibers, polypropylene fibers and polyethylene fibers. More preferably, a non-woven fabric is used since its density can be relatively easily controlled; it can be simply produced; and it is inexpensive.

As the porous resin layer, an organic polymer membrane may be preferably used. Examples of the material of the organic polymer membrane include polyethylene resins, polypropylene resins, polyvinyl chloride resins, polyvinylidene fluoride resins, polysulfone resins, polyethersulfone resins, polyacrylonitrile resins, cellulose resins and cellulose triacetate resins. The organic polymer membrane may be a mixture of resins containing one or more of these resins as the major component. The major component herein means that the component is contained in an amount of not less than 50% by weight, preferably not less than 60% by weight. Preferred examples of the material of the organic polymer membrane include those which can be easily formed by solutions and are excellent in physical durability and chemical resistance, such as polyvinyl chloride resins, polyvinylidene fluoride resins, polysulfone resins, polyethersulfone resins and polyacrylonitrile resins. A polyvinylidene fluoride resin or a resin containing it as the major component is most preferably used.

As the polyvinylidene fluoride resin, a homopolymer of vinylidene fluoride is preferably used. Further, as the polyvinylidene fluoride resin, a copolymer with vinyl monomers capable of copolymerizing with vinylidene fluoride is also preferably used. Examples of the vinyl monomers capable of copolymerizing with vinylidene fluoride include tetrafluoroethylene, hexafluoropropylene and ethylene fluoride trichloride.

The porous membrane that may be used as the separation membrane in the present invention is not limited as long as the microorganism(s) used for fermentation cannot pass through the membrane, and the membrane is preferably selected within the range in which secretions from the microorganism(s) used in the fermentation or particles in the fermentation feedstock do not cause clogging and the filtration performance is stably maintained for a long period. Therefore, the average pore size of the porous separation membrane is preferably not less than 0.01 $\mu$m and less than 5 $\mu$m. The average pore size is more preferably not less than 0.01 $\mu$m and less than 1 $\mu$m since, within this range, both a high blocking performance which does not allow leakage of microorganisms and a high permeability can be achieved, and the permeability can be maintained with higher accuracy and reproducibility for a long time.

In cases where the pore size is close to the size of the microorganism(s), the pores may be blocked by the microorganism(s). Therefore, the average pore size of the porous membrane is preferably less than 1 $\mu$m. In order to prevent leakage of the microorganism(s), that is, a decrease in the elimination rate of the microorganism(s), the average pore size of the porous membrane is preferably not too large as compared to the size of the microorganism(s). In cases where a microorganism having a small cell size such as a bacterium is used, the average pore size is preferably not more than 0.4 $\mu$m, more preferably less than 0.2 $\mu$m.

In some cases, the microorganism(s) may produce substances other than the chemical product of interest, e.g. substances that are likely to aggregate such as proteins and polysaccharides. Further, in some cases, death of a part of the microorganism(s) in the culture liquid may produce cell debris. In order to prevent clogging of the porous membrane due to these substances, the average pore size is still more preferably not more than 0.1 $\mu$m.

In cases where the average pore size is too small, the permeability of the porous membrane decreases, and thus an efficient operation cannot be carried out even with a clean membrane. Therefore, the average pore size of the porous membrane in the present invention is preferably not less than 0.01 $\mu$m, more preferably not less than 0.02 $\mu$m, still more preferably not less than 0.04 $\mu$m.

The average pore size can be determined by measuring the diameters of all pores which can be observed within an area of 9.2 $\mu$m $\times$ 10.4 $\mu$m under a scanning electron microscope at a magnification of 10,000$\times$, and then averaging the measured values. Alternatively, the average pore size can be determined by taking a picture of the membrane surface under a scanning electron microscope at a magnification of 10,000$\times$, and randomly selecting not less than 10, preferably not less than 20 pores, followed by measuring the diameters of these pores and calculating the number average. In cases where a pore is not circular, its size is determined by a method in which a circle whose area is equal to the area of the pore (equivalent circle) is determined using an image processing device or the like and then the diameter of the equivalent circle is regarded as the diameter of the pore.

The standard deviation $\sigma$ of the average pore size of the porous membrane used in the present invention is preferably not more than 0.1 $\mu$m. The standard deviation $\sigma$ of the average pore size is preferably as small as possible. The standard deviation $\sigma$ of the average pore size is calculated according to the (Equation 1) below, wherein N represents the number of pores observable within the above-mentioned area of 9.2 $\mu$m $\times$ 10.4 $\mu$m, $X_k$ represents the respective measured diameters, and X(ave) represents the average of the pore diameter.

[Equation 1]

$$\sigma = \sqrt{\frac{\sum_{k=1}^{N}\left(X_k - X(ave)\right)^2}{N}} \quad \cdots (1)$$

In the porous membrane used in the present invention, permeability to the fermentation culture liquid is one of the important performances. As an index of the permeability, the pure water permeability coefficient of the porous membrane before use can be employed. In the present invention, the pure water permeability coefficient of the porous membrane is preferably not less than $5.6 \times 10^{-10}$ m$^3$/m$^2$/s/pa when calculated by measuring the amount of permeation of water with a head height of 1 m using purified water at a temperature of 25°C prepared with a reverse osmosis membrane. In cases where the pure water permeability coefficient is from $5.6 \times 10^{-10}$ m$^3$/m$^2$/s/pa to $6 \times 10^{-7}$ m$^3$/m$^2$/s/pa, an amount of permeation which is practically sufficient can be obtained.

In the porous membrane used in the present invention, the surface roughness is the average of the height in the direction vertical to the surface. The membrane surface roughness is a factor that influences how easily a microorganism attached to the surface of a separation membrane is detached by the effect of washing the membrane surface with flowing liquid generated by stirring or a circulating pump. The surface roughness of the porous membrane is not limited as long as it is within the range in which the microorganism(s) and other solids attached to the membrane can be detached. The surface roughness is preferably not more than 0.1 $\mu$m. In cases where the surface roughness is not more than 0.1 $\mu$m, the microorganism(s) and other solids attached to the membrane can be easily detached.

It was found that an operation that does not require excessive power for washing the membrane surface can be carried out more easily by using, more preferably, a porous membrane having a membrane surface roughness of not more than 0.1 $\mu$m, an average pore size of not less than 0.01 $\mu$m and less than 1 $\mu$m, and a pure water permeability coefficient of not less than $2 \times 10^{-9}$ m$^3$/m$^2$/s/pa. In cases where the surface roughness of the porous membrane is not more than 0.1 $\mu$m, the shear force generated on the membrane surface during filtration of the microorganism(s) can be reduced, and hence destruction of the microorganism(s) can be suppressed, and clogging of the porous membrane can also be suppressed. Thus, long-time stable filtration can be more easily carried out. Further, in cases where the surface roughness of the porous membrane is not more than 0.1 $\mu$m, continuous fermentation can be carried out with a smaller transmembrane pressure difference. Therefore, even in cases where clogging of the porous membrane has occurred, a better washing recovery performance can be obtained as compared to cases where the operation was carried out with a larger transmembrane pressure difference. Since suppression of clogging of the porous membrane allows stable continuous fermentation, the surface roughness of the porous membrane is preferably as small as possible.

The membrane surface roughness of the porous membrane herein is measured using the following atomic force microscope (AFM) under the following conditions.

Device

Atomic force microscope ("Nanoscope IIIa", manufactured by Digital Instruments, Inc.)

• Conditions

Probe: SiN cantilever (manufactured by Digital Instruments, Inc.)
Scanning mode:

Contact mode (measurement in air)
Underwater tapping mode (underwater measurement)

Scanning area:

10 $\mu$m $\times$ 25 $\mu$m (measurement in air)
5 $\mu$m $\times$ 10 $\mu$m (underwater measurement)

Scanning resolution: 512 $\times$ 512

• sample preparation

When the measurement was carried out, the membrane sample was soaked in ethanol at room temperature for 15 minutes and then soaked in RO water for 24 hours to wash it, followed by drying in the air. The RO water means water prepared by filtration through a reverse osmosis membrane (RO membrane), which is a type of filtration membrane, to remove impurities such as ions and salts. The pore size of the RO membrane is not more than about 2 nm.

The membrane surface roughness $d_{rough}$ was calculated according to the Equation (2) below based on the height of each point in the direction of the Z-axis, as determined using the atomic force microscope (AFM).
[Equation 2]

$$d_{rough} = \sum_{n=1}^{N} \frac{|Z_n - \overline{Z}|}{N} \quad \cdots (2)$$

$d_{rough}$ :Average surface roughness ($\mu$m)
$Z_n$ :Height in the direction of the Z-axis ($\mu$m)
$\overline{Z}$ :Average height in the scanned area ($\mu$m)

The shape of the porous membrane used in the present invention is preferably a flat membrane. In cases where the shape of the porous membrane is a flat membrane, its average thickness is selected depending on its use. The average thickness in the cases where the shape of the porous membrane is a flat membrane is preferably 20 $\mu$m to 5000 $\mu$m, more preferably 50 $\mu$m to 2000 $\mu$m. Further, the shape of the porous membrane used in the present invention is preferably a hollow fiber membrane. In cases where the porous membrane is a hollow fiber membrane, the inner diameter of the hollow fiber is preferably 200 $\mu$m to 5000 $\mu$m, and the membrane thickness is preferably 20 $\mu$m to 2000 $\mu$m. A fabric or knit produced by forming organic fibers or inorganic fibers into a cylindrical shape may be contained in the hollow fiber.

The porous membrane described above can be produced by, for example, the production method described in WO2007/097260.

In another preferred embodiment, the separation membrane in the present invention may be a membrane containing at least a ceramic. The ceramic in the present invention means a substance that contains a metal oxide and was baked by heat treatment at high temperature. Examples of the metal oxide include alumina, magnesia, titania and zirconia. The separation membrane may be formed by only a metal oxide(s), or may contain silica and/or silicon carbide, and/or mullite and/or cordierite, which are compounds of silica and a metal oxide(s).

Components forming the separation membrane other than the ceramic are not limited as long as the components can form a porous body as a separation membrane.

Even in cases where the separation membrane contains a ceramic, the shape of the separation membrane is not limited, and may be any of a monolith membrane, flat membrane, tubular membrane and the like. In view of the efficiency of packing into a container, the separation membrane preferably has a columnar shape in which a penetrating hole(s) is/are formed in the longitudinal direction. In view of increasing the packing efficiency, the separation membrane is preferably a monolith membrane.

The reason why the separation membrane preferably has a penetrating hole(s) in the longitudinal direction is as follows. In cases where a separation membrane having a columnar structure is placed in a modular container to use it as a separation membrane module, modularization of the separation membrane is possible by selecting a preferred mode from the external-pressure type and the internal-pressure type, and filtration can be carried out with the module. In the present invention, the side in which the separation membrane contacts with the fermentation culture liquid is hereinafter referred to as the primary side, and the side in which a filtrate containing a chemical product is obtained by filtration is hereinafter referred to as the secondary side.

In cases where a inner-pressure type module is used, the channel in the primary side is narrow. Therefore, the output of the circulating pump during cross-flow filtration can be saved. Further, the action to discharge the suspended matter accumulated on the surface of the separation membrane is strong, and therefore the surface of the separation membrane is likely to be kept clean, which is preferred. However, in order to obtain this effect, the inner-pressure type separation membrane needs to have an inlet and an outlet for the fermentation culture liquid. The inlet and the outlet are preferably in a state where they are arranged on a straight line to form a penetrating hole since the flow resistance is small in such a case. Further, in cases where the separation membrane has a columnar shape and the penetrating hole(s) open(s) in the longitudinal direction, the container containing the separation membrane can be made thin. A thin separation membrane module is preferred in view of production and handling.

The porosity of the separation membrane is not limited, but in cases where the porosity is too low, the filtration efficiency is low; and in cases where the porosity is too high, the strength is low. In order to achieve both high filtration efficiency and high strength of the separation membrane, as well as resistance to repeated steam sterilization, the porosity is preferably 20% to 60%.

The porosity is determined according to the following equation.

Porosity [%] = 100 × (wet membrane weight [g] - dry membrane weight [g]) /

specific gravity of water [g/cm$^3$] / (membrane volume [cm$^3$])

The average pore size of the separation membrane is preferably 0.01 $\mu$m to 1 $\mu$m, and a membrane having an average pore size within this range is less likely to be clogged and has excellent filtration efficiency. Further, with an average pore size within the range of 0.02 $\mu$m to 0.2 $\mu$m, substances that easily cause clogging of a separation membrane, such as by-products of fermentation by the microorganism or cultured cells, including proteins and polysaccharides, and cell debris produced by death of the microorganism/cultured cells in the culture liquid, become less likely to cause clogging, which is especially preferred.

In a separation membrane having a penetrating hole(s) and a columnar structure, the outer surface is in the secondary side. Therefore, it is preferred that a modular container be provided for collecting the filtrate and that the separation membrane be packed into the container to form a module to be used. One or more separation membranes are packed into one module.

The modular container is preferably composed of a material resistant to repeated steam sterilization. Examples of the material resistant to steam sterilization include stainless steels, and ceramics having low average porosities.

Such a ceramic membrane module can be produced by, for example, the production method described in WO2012/086763, or a commercially available module may be used. Specific examples of the commercially available module include MEMBRALOX Microfiltration Membrane (Pall Corporation) and a ceramic membrane filter Cefilt MF Membrane (NGK Insulators, Ltd.).

The continuous fermentation is explained below.

The continuous fermentation in the present invention is characterized in that it is continuous fermentation in which a culture liquid of a microorganism(s) is filtered through a separation membrane; unfiltered liquid is retained in, or refluxed to, the culture liquid; a fermentation feedstock is added to the culture liquid; and a product is recovered from the filtrate.

In the culture of a microorganism(s), a pH and a temperature suitable for the microorganism(s) used may be set, and the pH and the temperature are not limited as long as the microorganism(s) can be grown. The pH and the temperature are usually within the ranges of 4 to 8 and 20 to 75°C, respectively. The pH of the culture liquid is adjusted in advance to a predetermined value usually within the range of 4 to 8 using an inorganic or organic acid, alkaline substance, urea, calcium carbonate, ammonia gas or the like.

The sugar concentration in the culture liquid in the continuous fermentation is preferably kept at not more than 5 g/L in view of productivity. The sugar concentration in the culture liquid can be controlled by the rate of supplying the culture medium or the rate of filtering the culture liquid during the continuous fermentation, or by the sugar concentration in the culture medium to be supplied. Another means for controlling the sugar concentration is to control the amount of oxygen supplied.

The pentose metabolism by the microorganism(s) having pentose reductase and pentose dehydrogenase used in the present invention often proceeds more advantageously as the oxygen concentration increases. It is known that ethanol fermentation and the like using glucose usually proceed more efficiently when such fermentations are carried out under anaerobic conditions, in which oxygen is not utilized. However, pentose metabolism proceeds more advantageously under aerobic conditions. It is thought that this is because most of pentose reductases utilize NADH as a coenzyme, and oxidation and reduction are imbalanced in the pentose metabolic pathway, which imbalance may cause the excessive use of the pathway using oxygen. Some microorganisms have a pentose reductase that is a dual enzyme capable of utilizing both NADH and NADPH. Such an enzyme can prevent the imbalance in the NAD/NADH oxidization/reduction system under conditions where oxygen is limited (Metabolic Engineering: Principles and Methodologies. Gregory N. Stephanopoulos et al., Tokyo Denki University Press. p. 174 (2002)). Therefore, the amount of oxygen supplied needs to be set appropriately depending on the microorganism(s) used. Even with such a dual enzyme, NADH is more likely to be used than NADPH, and, in such cases, the metabolism is possible also under aerobic conditions. Therefore, as for the oxygen condition in the culture in the present invention, the oxygen transfer coefficient KLa (hr$^{-1}$) (hereinafter simply referred to as KLa) is preferably 5 to 300 hr$^{-1}$, more preferably 10 to 250 hr$^{-1}$, still more preferably 20 to 200 hr$^{-1}$. In cases where KLa is not more than 5 hr$^{-1}$, the production efficiency may be low since the sugar consumption rate is low. In cases where KLa is not less than 300 hr$^{-1}$, excessive foaming may interfere with the continuous fermentation.

KLa represents the capacity to produce dissolved oxygen by transferring oxygen from the gas phase to the liquid phase in a unit time with aeration and stirring, and is defined by the Equation (3) below. (Laboratory Manual for Bioengineering. The Society for Biotechnology, Japan ed., Baifukan Co., Ltd., p. 310 (1992)).

$$dC/dt = KLa \times (C^*\text{-}C) \dots (3)$$

In this equation, C represents the dissolved oxygen level DO (ppm) in the culture liquid; C* represents the dissolved oxygen level DO (ppm) in the state of equilibration with the gas phase in the absence of consumption of oxygen by the microorganism(s); and KLa represents the oxygen transfer coefficient (hr$^{-1}$). Since the Equation (4) below is derived from the Equation (3) above, KLa can be determined by plotting the logarithm of C*-C against the period of aeration.

$$\ln(C^*-C) = -KLa \times t \dots (4)$$

KLa in the present invention is a value measured by the gassing-out method (dynamic method). The gassing-out method (dynamic method) means a method in which water or the culture medium to be used is placed in an aeration-stirring culture apparatus comprising a dissolved-oxygen concentration electrode inserted therein, and oxygen in the liquid is replaced by nitrogen gas to decrease the oxygen concentration in the liquid, followed by exchanging the nitrogen gas with compressed air and measuring the process of increase of dissolved oxygen at a predetermined aeration rate, stirring rate and temperature, to calculate KLa.

KLa can be set appropriately by combining an aeration condition and a stirring condition to perform culture with aeration and stirring. The gas to be used for aeration in such a case may be changed depending on culture conditions. High dissolved oxygen level can be kept by, for example, maintaining the oxygen concentration at not less than 21 % by adding oxygen into the air, pressurizing the culture liquid, increasing the stirring rate, and/or increasing the aeration rate.

KLa in the present invention may be determined by direct measurement by the above-described means. Alternatively, in cases where KLa is within a low range, it may be determined by calculation since KLa is known to be proportional to the aeration rate as shown by the Equation (5) below at a constant stirring rate. More specifically, KLa is measured at a constant stirring rate while the aeration rate is arbitrarily changed, and the obtained KLa values are plotted against the aeration rate, followed by determining the constants a and b that satisfy Equation (5), so that KLa in the present invention can be set.

$$KLa = a \times V + b \dots (5)$$

In this equation, V represents the aeration rate (vvm); and a and b are constants. In cases where KLa is too high to satisfy Equation (5), direct measurement is carried out to determine KLa.

In the method for producing the chemical product of the present invention, the transmembrane pressure difference during filtration is not limited as long as the fermentation culture liquid can be filtered. However, in cases where filtration treatment is carried out through an organic polymer membrane with a transmembrane pressure difference of more than 150 kPa, the structure of the organic polymer membrane is highly likely to be destroyed, and therefore the capacity to produce chemical products may be deteriorated. In cases where the transmembrane pressure difference is less than 0.1 kPa, a sufficient amount of permeate of the fermentation culture liquid may not be obtained, and the productivity in production of the chemical product tends to be low. When an organic polymer membrane is used in the method for producing a chemical product of the present invention, the transmembrane pressure difference, which is the filtration pressure, is preferably within the range of 0.1 kPa to 150 kPa since, in such a case, the amount of permeate of the fermentation culture liquid can be large, and the decrease in the capacity to produce a chemical product due to destruction of the membrane structure does not occur. Therefore, the capacity to produce a chemical product can be kept high in such a case. In cases of an organic polymer membrane, the transmembrane pressure difference is more preferably within the range of 0.1 kPa to 50 kPa, still more preferably within the range of 0.1 kPa to 20 kPa.

Also in cases where a ceramic membrane is used, the transmembrane pressure difference during filtration is not limited as long as the fermentation culture liquid can be filtered. The transmembrane pressure difference is preferably not more than 500 kPa. In cases where the operation is carried out at not less than 500 kPa, clogging of the membrane may occur to cause a trouble in the operation of continuous fermentation.

In terms of the driving force for the filtration, a siphon using the liquid level difference (hydraulic head difference) between the fermentation culture liquid and the liquid processed through the porous membrane, or a cross-flow circulating pump, may be used to generate the transmembrane pressure difference in the separation membrane. Further, as the driving force for the filtration, a suction pump may be placed in the secondary side of the separation membrane. In cases where a cross-flow circulating pump is used, the transmembrane pressure difference can be controlled by the suction pressure. The transmembrane pressure difference can also be controlled by the pressure of the gas or liquid which is used for introducing the pressure into the fermentation liquid side. In cases where such pressure control is carried out, the difference between the pressure in the fermentation liquid side and the pressure in the side of the liquid processed through the porous membrane can be regarded as the transmembrane pressure difference, and can be used for controlling the transmembrane pressure difference.

In the method for producing a chemical product of the present invention, continuous fermentation (filtration of culture liquid) may be started after increasing the microorganism concentration by performing batch culture or fed-batch culture at an early stage of culture. Alternatively, microorganism cells may be seeded at high concentration, and continuous fermentation may then be carried out from the beginning of the culture. In the method for producing a chemical product of the present invention, supply of the culture medium and filtration of the culture liquid may be carried out from an appropriate timing(s). The timings of beginning of the supply of the culture medium and filtration of the culture liquid do not necessarily need to be the same. The supply of the culture medium and filtration of the culture liquid may be carried out either continuously or intermittently.

A nutrient(s) necessary for growth of the microorganism cells may be added to the culture medium to allow continuous growth of the cells. The microorganism concentration in the culture liquid is not limited as long as it is a concentration preferred for efficient production of the chemical product. A good production efficiency can be obtained by maintaining the microorganism concentration in the culture liquid at, for example, not less than 5 g/L in terms of the dry weight.

If necessary, during the continuous fermentation in the method for producing a chemical product of the present invention, the microorganism concentration in the culture vessel may be controlled by removing a part of the culture liquid containing the microorganism(s) from the fermenter and then diluting the culture liquid in the vessel with a culture medium. For example, when the microorganism concentration in the fermenter is too high, clogging of the separation membrane is likely to occur. The clogging of the separation membrane can be avoided by removing a part of the culture liquid containing the microorganism(s) and then diluting the culture liquid in the fermenter with the culture medium. Further, the performance for producing the chemical product may change depending on the microorganism concentration in the fermenter. The production performance may be maintained by removing a part of the culture liquid containing the microorganism(s) and then diluting the culture liquid in the fermenter with a culture medium, using the production performance as an index.

In cases where continuous fermentation is carried out according to the method for producing a chemical product of the present invention, the continuous fermentation can be performed with an extremely higher fermentation yield as compared to cases where conventional culture is performed. The fermentation yield in continuous fermentation herein is calculated according to the Equation (6) below.

$$\text{Yield (g/g)} = \text{amount of product (g)} / \{\text{fed sugar (g)} - \text{unused sugar (g)}\} \ldots (6)$$

The continuous fermentation apparatus used in the present invention is not limited as long as it is an apparatus for producing a chemical product by continuous fermentation in which a fermentation culture liquid of a microorganism(s) is filtered through a separation membrane and the product is recovered from the filtrate, while the unfiltered liquid is retained in, or refluxed to, the fermentation culture liquid; a fermentation feedstock is added to the fermentation culture liquid; and the product in the filtrate is recovered. Specific examples of the apparatus in which an organic polymer membrane is used include the apparatus described in WO2007/097260. Specific examples of the apparatus in which a ceramic membrane is used include the apparatus described in WO2012/086763.

The chemical product produced by the method for producing a chemical product of the present invention is not restricted as long as it is a substance produced in a culture liquid by the above-described microorganisms. Examples of the chemical product produced by the method for producing a chemical product of the present invention include alcohols, organic acids, amino acids and nucleic acids, which are substances mass-produced in the fermentation industry. Examples the substances include alcohols such as ethanol, isobutanol, isopropanol, n-butanol, 1,3-propanediol, 1,4-butanediol, 2,3-butanediol and glycerol; organic acids such as acetic acid, lactic acid, pyruvic acid, succinic acid, malic acid, itaconic acid, citric acid and adipic acid; nucleic acids such as nucleosides including inosine and guanosine and nucleotides including inosinic acid and guanylic acid; and diamine compounds such as cadaverine and putrescine. Further, the present invention may also be applied to production of substances such as enzymes, antibiotics and recombinant proteins. These chemical products can be recovered from the filtrate by well-known methods (membrane separation, concentration, distillation, crystallization, extraction and the like).

EXAMPLES

The present invention will now be described concretely by way of Examples. However, the present invention is not limited to these.

(Reference Example 1) Method for Analyzing Glucose, Xylose and Ethanol

The concentrations of glucose, xylose and ethanol in the culture liquid were quantified under the following HPLC conditions by comparison with standard samples.

Column: Shodex SH1011 (manufactured by Showa Denko K. K.)
Mobile phase: 5 mM sulfuric acid (flow rate: 0.6 mL/min)
Reaction liquid: none
Detection method: RI (differential refractive index)
Temperature: 65 °C

(Reference Example 2) Method for Analyzing Lactic Acid

Lactic acid in the culture liquid was quantified under the following HPLC conditions by comparison with standard samples.

Column: Shim-Pack SPR-H (manufactured by Shimadzu Corporation)
Mobile phase: 5 mM p-toluenesulfonic acid (flow rate: 0.8 mL/min)
Reaction liquid: 5 mM p-toluenesulfonic acid, 20 mM Bis-Tris, 0.1 mM EDTA-2Na (flow rate: 0.8 mL/min)
Detection method: electric conductivity
Temperature : 45°C

(Reference Example 3) Measurement of Pentose Reductase Activity

Each of the *Candida tropicalis* NBRC0199 strain, the *Pichia stipitis* NBRC1687 strain, and the *Candida utilis* CuLpLDH strain described in WO2010/140602 was inoculated to 5 mL of YPX medium (1% yeast extract, 2% bactopeptone, 2% xylose) using a platinum loop, and culture was carried out at 30°C overnight with shaking (preculture). On the next day, the preculture liquid was inoculated to 50 mL of YPX medium placed in a 500-mL baffled Erlenmeyer flask, and culture was performed at 30°C for 24 hours with shaking. The collected culture liquid was washed twice with 50 mM phosphate buffer. The microorganism cells were resuspended in 50 mM phosphate buffer, and homogenized with a bead homogenizer (0.6 g of φ0.5-beads, 4000 rpm, 1 minute × 5 times), followed by centrifugation and recovery of the resulting supernatant, which supernatant was used as a microorganism cell extract. The microorganism cell extract was added to 50 mM phosphate buffer (900 μL) supplemented with 200 mM xylose and 100 μL of 1.5 mM NAD(P)H, and the reaction was allowed to proceed at 30°C while the absorbance at 340 nm was continuously monitored. A sample without addition of xylose was used as a blank. In cases where the extent of decrease in the absorbance was larger than that of the blank, the strain was judged to have a pentose reductase activity. As a result, all of the 3 strains could be confirmed to have a pentose reductase activity.

(Reference Example 4) Measurement of Pentol Dehydrogenase Activity

Each of the *Candida tropicalis* NBRC0199 strain, the *Pichia stipitis* NBRC1687 strain, and the *Candida utilis* CuLpLDH strain described in WO2010/140602 was inoculated to 5 mL of YPX medium (1% yeast extract, 2% bactopeptone, 2% xylose) using a platinum loop, and culture was carried out at 30°C overnight with shaking (preculture). On the next day, the preculture liquid was inoculated to 50 mL of YPX medium placed in a 500-mL baffled Erlenmeyer flask, and culture was performed at 30°C for 24 hours with shaking. The collected culture liquid was washed twice with 50 mM Tris-HCl buffer. The microorganism cells were resuspended in 50 mM Tris-HCl buffer, and homogenized with a bead homogenizer (0.6 g of φ0.5-beads, 4000 rpm, 1 minute × 5 times), followed by centrifugation and recovery of the resulting supernatant, which supernatant was used as a microorganism cell extract. The microorganism cell extract was added to 900 μL of 50 mM Tris-HCl buffer supplemented with 50 mM MgCl$_2$, 300 mM xylitol and 100 μL of 10 mM NAD(P)+, and the reaction was allowed to proceed at 35°C while the absorbance at 340 nm was continuously monitored. A sample without addition of xylitol was used as a blank. In cases where the extent of increase in the absorbance was larger than that of the blank, the strain was judged to have a pentol dehydrogenase activity. As a result, all of the 3 strains could be confirmed to have a pentol dehydrogenase activity.

(Reference Example 5) Measurement of KLa

A dissolved-oxygen electrode (manufactured by Mettler-Toledo) was inserted into the culture vessel to measure the dissolved-oxygen level under each aeration/stirring condition, and KLa was determined by the dynamic method using nitrogen gas. In a culture vessel, 1.5 L of water was placed, and nitrogen gas was sufficiently blown into the water while the water temperature was controlled at 30°C and the water was stirred at a constant rate. When the electrode value became minimum, zero calibration of the dissolved-oxygen electrode was carried out. Thereafter, the aeration gas was changed from nitrogen gas to air at a predetermined aeration rate, and changes in the dissolved-oxygen level with time were measured to determine KLa. Table 1 shows KLa obtained for various aeration/stirring conditions at a stirring rate of 800 rpm and a temperature of 30°C using compressed air.

[Table 1]

| Aeration gas | Aeration rate (vvm) | Stirring rate (rpm) | 30°C |
| --- | --- | --- | --- |
| | | | KLa (hr$^{-1}$) |
| Air | 0.01 | 800 | 4 |
| | 0.05 | 800 | 14 |
| | 0.1 | 800 | 27 |
| | 0.2 | 800 | 57 |
| | 0.3 | 800 | 89 |
| | 0.5 | 800 | 144 |
| | 0.6 | 800 | 168 |
| | 1.5 | 800 | 327 |

The relationship between the aeration rate and KLa was plotted within the range of 0.01 to 0.6 wm in Table 1 to calculate the constants (a, b) in Equation (5). As a result, the constants (a, b) were (284, 0.49). Since the value for 1.5 vvm did not satisfy the linear relationship of the plot, the value was not included in the calculation of constants.

(Comparative Example 1) Production of Ethanol by Batch Culture of *Candida tropicalis* Using Hexose as Fermentation Feedstock

The *Candida tropicalis* NBRC0199 strain was used as the microorganism, and YPD medium (1% yeast extract, 2% bactopeptone, 7% glucose) was used as the culture medium. The *Candida tropicalis* NBRC0199 strain was cultured in 2 mL of YPD medium in a test tube at 30°C overnight with shaking (pre-preculture). The obtained culture liquid was inoculated to 50 mL of YPD medium placed in a 500-mL baffled Erlenmeyer flask, and culture was performed overnight with shaking (preculture). The preculture liquid was inoculated to 1.5 L of YPD medium, and batch culture was performed for 16 hours under the following conditions with stirring at 800 rpm by the stirrer attached to the culture vessel while the aeration rate and the temperature in the culture vessel were controlled, to produce ethanol (Table 3).

Culture vessel capacity: 2 L
Effective capacity of the culture vessel: 1.5 L
Temperature adjustment: 30 (°C)
Aeration rate in the culture vessel: 0.1 vvm, compressed air
Stirring rate in the culture vessel: 800 rpm
pH Adjustment: none
Sterilization: culture vessels and media to be used were all subjected to high-pressure steam sterilization by autoclaving at 121 °C for 20 min.

(Comparative Example 2) Production of Ethanol by Batch Culture of *Candida tropicalis* Using Mixed Sugar as Fermentation Feedstock

Using the YPDX medium having the composition shown in Table 2 as the culture medium, batch culture was performed for 23 hours under the same conditions as in Comparative Example 1, to produce ethanol (Table 3).

[Table 2]

| Components | Content |
| --- | --- |
| Glucose | 3% |
| Xylose | 4% |
| Bactopeptone | 2% |
| Yeast extract | 1% |

(Comparative Example 3) Production of Ethanol by Continuous Fermentation by *Candida tropicalis* Using Mixed Sugar as Fermentation Feedstock

Using the YPDX medium having the composition shown in Table 2 as the culture medium, continuous fermentation was carried out without using a separation membrane. The *Candida tropicalis* NBRC0199 strain was cultured in 2 mL of YPD medium in a test tube at 30°C overnight with shaking (pre-pre-preculture). The obtained culture liquid was inoculated to 50 mL of YPD medium placed in a 500-mL baffled Erlenmeyer flask, and culture was performed overnight with shaking (pre-preculture). The pre-preculture liquid was inoculated to 1.5 L of YPDX medium placed in a continuous fermentation apparatus (the same apparatus as shown in Fig. 2 of WO2007/097260 except that the separation membrane element was eliminated), and culture was performed for 16 hours under the same conditions for the stirring rate, aeration rate and temperature in the culture vessel as in Comparative Example 1 (preculture). Immediately after completion of the preculture, continuous culture was performed for 360 hours to produce ethanol. For supplying the culture medium and collecting the culture liquid containing the microorganism, a Perista BioMini Pump Type AC-2120 (ATTO) was used to supply the culture medium directly to the culture vessel and to collect the culture liquid containing the microorganism directly from the culture vessel. Setting the rate of collection of the culture liquid containing the microorganism to 0.05 L/hr, the operation was carried out while the rate of supplying the culture medium was controlled such that the amount of culture liquid in the culture vessel was 1.5 L (Table 3).

(Example 1) Production of Ethanol by Continuous Fermentation by *Candida tropicalis* Using Mixed Sugar as Fermentation Feedstock, with Use of Separation Membrane 1

Using the YPDX medium having the composition shown in Table 2 as the culture medium, continuous fermentation was carried out with use of a separation membrane. The *Candida tropicalis* NBRC0199 strain was cultured in 2 mL of YPD medium in a test tube at 30°C overnight with shaking (pre-pre-preculture). The obtained culture liquid was inoculated to 50 mL of YPD medium placed in a 500-mL baffled Erlenmeyer flask, and culture was performed overnight with shaking (pre-preculture). The pre-preculture liquid was inoculated to 1.5 L of YPDX medium placed in a continuous culture apparatus equipped with an integrated membrane having the properties shown below (the apparatus shown in Fig. 2 of WO2007/097260), and culture was performed for 36 hours with stirring at 800 rpm by the stirrer attached to the culture vessel while the aeration rate and the temperature in the culture vessel were controlled (preculture). Immediately after completion of the preculture, continuous fermentation was started to produce ethanol. For supplying the culture medium and filtering the culture liquid, a Perista BioMini Pump Type AC-2120 (ATTO) was used. The culture medium was directly supplied to the culture vessel, and filtration of the culture liquid was carried out by collecting the filtrate through an element having a separation membrane fixed thereto. While the rate of supplying the culture medium was controlled such that the amount of culture liquid in the culture vessel was 1.5 L at a constant rate of filtration of the culture liquid, and while the transmembrane pressure difference during filtration was allowed to change within the range of 0.1 to 20.0 kPa, continuous fermentation was performed for 370 hours to produce ethanol (Table 3).

Culture vessel capacity: 2 L
Effective capacity of the culture vessel: 1.5 L
Separation membrane used: polyvinylidene fluoride filtration membrane
Effective filtration area of the membrane separation element: 120 cm$^2$
Pure water permeation coefficient of the separation membrane: $50 \times 10^{-9}$ m$^3$/m$^2$/s/Pa
Average pore size of the separation membrane: 0.1 $\mu$m
Standard deviation of the average pore size: 0.035 $\mu$m
Surface roughness of the separation membrane: 0.06 $\mu$m
Temperature adjustment: 30 (°C)
Aeration rate in the culture vessel: 0.01 vvm, compressed air
Stirring rate in the culture vessel: 800 rpm
pH Adjustment: none
Rate of filtration of the culture liquid: 0.05 L/hr
Sterilization: the culture vessels comprising the separation membrane element and media to be used were all subjected to high-pressure steam sterilization by autoclaving at 121 °C for 20 min.

(Example 2) Production of Ethanol by Continuous Fermentation by *Candida tropicalis* Using Mixed Sugar as Fermentation Feedstock, with Use of Separation Membrane 2

Using the *Candida tropicalis* NBRC0199 strain, continuous fermentation was carried out with use of a separation membrane. The continuous fermentation was carried out for 369 hours under the same conditions as in Example 1 except that the rate of aeration with compressed air in the culture vessel was 0.1 μm, to produce ethanol (Table 3).

(Example 3) Production of Ethanol by Continuous Fermentation by *Candida tropicalis* Using Mixed Sugar as Fermentation Feedstock, with Use of Separation Membrane 3

Using the *Candida tropicalis* NBRC0199 strain, continuous fermentation was carried out with use of a separation membrane. The continuous fermentation was carried out for 370 hours under the same conditions as in Example 1 except that the rate of aeration with compressed air in the culture vessel was 1.5 wm, to produce ethanol (Table 3).

[Table 3]

| | Comparative Example 1: batch fermentation | Comparative Example 2: batch fermentation | Comparative Example 3: continuous fermentation | Example 1: continuous fermentation using membrane | Example 2: continuous fermentation using membrane | Example 3: continuous fermentation using membrane |
|---|---|---|---|---|---|---|
| Fermentation period (hr) | 16 | 23 | 360 | 370 | 369 | 370 |
| Total glucose fed (g) | 106 | 48.0 | 568 | 594 | 613 | 620 |
| Total xylose fed (g) | 0 | 64.1 | 738 | 742 | 827 | 783 |
| Unused glucose (g) | 0 | 0 | 0 | 0 | 0 | 0 |
| Unused xylose (g) | 0 | 0 | 72 | 37 | 18 | 6 |
| Total production of ethanol (g) | 48.6 | 26.9 | 333 | 429 | 483 | 433 |
| Ethanol yield (g/g) | 0.46 | 0.24 | 0.27 | 0.33 | 0.34 | 0.31 |

(Comparative Example 4) Production of Ethanol by Batch Culture of *Pichia stipitis* Using Hexose as Fermentation Feedstock

Using the *Pichia stipitis* NBRC1687 strain as the microorganism, batch culture was performed for 40 hours under the same conditions as in Comparative Example 1 to produce ethanol (Table 4).

(Comparative Example 5) Production of Ethanol by Batch Culture of *Pichia stipitis* Using Mixed Sugar as Fermentation Feedstock

Using the *Pichia stipitis* NBRC1687 strain as the microorganism, batch culture was performed for 40 hours under the same conditions as in Comparative Example 2 to produce ethanol (Table 4).

(Comparative Example 6) Production of Ethanol by Continuous Fermentation by *Pichia stipitis* Using Mixed Sugar as Fermentation Feedstock

Using the *Pichia stipitis* NBRC1687 strain as the microorganism, continuous fermentation was carried out with a mixed-sugar material, without use of a separation membrane. The continuous fermentation was performed for 368 hours under the same conditions as in Comparative Example 3 except that the period of preculture was 40 hours, to produce ethanol (Table 4).

(Example 4) Production of Ethanol by Continuous Fermentation by *Pichia stipitis* Using Mixed Sugar as Fermentation Feedstock, with Use of Separation Membrane 1

Using the *Pichia stipitis* NBRC1687 strain as the microorganism, continuous fermentation was carried out with use of a separation membrane. The continuous fermentation was performed for 370 hours under the same conditions as in Example 1 except that the period of preculture was 48 hours and that the transmembrane pressure difference was 0.1 to 19.8 kPa, to produce ethanol (Table 4).

(Example 5) Production of Ethanol by Continuous Fermentation by *Pichia stipitis* Using Mixed Sugar as Fermentation Feedstock, with Use of Separation Membrane 2

Using the *Pichia stipitis* NBRC1687 strain as the microorganism, continuous fermentation was carried out with use of a separation membrane. The continuous fermentation was performed for 369 hours under the same conditions as in Example 2 except that the period of preculture was 40 hours and that the transmembrane pressure difference was 0.1 to 19.8 kPa, to produce ethanol (Table 4).

(Example 6) Production of Ethanol by Continuous Fermentation by *Pichia stipitis* Using Mixed Sugar as Fermentation Feedstock, with Use of Separation Membrane 3

Using the *Pichia stipitis* NBRC1687 strain as the microorganism, continuous fermentation was carried out with use of a separation membrane. The continuous fermentation was performed for 370 hours under the same conditions as in Example 3 except that the period of preculture was 36 hours and that the transmembrane pressure difference was 0.1 to 19.8 kPa, to produce ethanol (Table 4).

[Table 4]

| | Comparative Example 4: batch fermentation | Comparative Example 5: batch fermentation | Comparative Example 6: continuous fermentation | Example 4: continuous fermentation using membrane | Example 5: continuous fermentation using membrane | Example 6: continuous fermentation using membrane |
|---|---|---|---|---|---|---|
| Fermentation period (hr) | 24 | 40 | 368 | 370 | 369 | 370 |
| Total glucose fed (g) | 105 | 48.0 | 581 | 557 | 613 | 583 |
| Total xylose fed (g) | 0 | 64.1 | 754 | 747 | 827 | 744 |
| Unused glucose (g) | 0 | 0 | 0 | 0 | 0 | 0 |
| Unused xylose (g) | 0 | 0 | 129 | 56 | 42 | 9 |
| Total production of ethanol (g) | 49.4 | 33.6 | 397 | 487 | 559 | 487 |
| Ethanol yield (g/g) | 0.47 | 0.30 | 0.33 | 0.39 | 0.40 | 0.37 |

(Comparative Example 7) Production of D-Lactic Acid by Batch Culture of *Candida utilis* Using Hexose as Fermentation Feedstock

As the microorganism, the *Candida utilis* CuLpLDH strain, which was prepared by the method disclosed in WO2010/140602, was used. Batch culture was carried out for 40 hours under the same conditions as in Comparative Example 1 except that the pH was adjusted to 6.0 with 1 N calcium hydroxide, to produce D-lactic acid (Table 5).

(Comparative Example 8) Production of D-Lactic Acid by Batch Culture of *Candida utilis* Using Mixed Sugar as Fermentation Feedstock

Batch culture was carried out for 40 hours under the same conditions as in Comparative Example 2 except that the *Candida utilis* CuLpLDH strain was used as the microorganism and that the pH was adjusted to 6.0 with 1 N calcium hydroxide, to produce D-lactic acid (Table 5).

(Comparative Example 9) Production of D-Lactic Acid by Continuous Fermentation by *Candida utilis* Using Mixed Sugar as Fermentation Feedstock

Continuous fermentation was carried out using the *Candida utilis* CuLpLDH strain as the microorganism, without use of a separation membrane. The continuous fermentation was carried out for 368 hours under the same conditions as in Comparative Example 3 except that the period of preculture was 40 hours and that the pH was adjusted to 6.0 with 1 N calcium hydroxide, to produce D-lactic acid (Table 5).

(Example 7) Production of D-Lactic Acid by Continuous Fermentation by *Candida utilis* Using Mixed Sugar as Fermentation Feedstock, with Use of Separation Membrane 1

Continuous fermentation was carried out using the *Candida utilis* CuLpLDH strain as the microorganism, with use of a separation membrane. The continuous fermentation was performed for 370 hours under the same conditions as in Example 1 except that the period of preculture was 50 hours and that the pH was adjusted to 6.0 with 1 N calcium hydroxide, to produce D-lactic acid (Table 5).

(Example 8) Production of D-Lactic Acid by Continuous Fermentation by *Candida utilis* Using Mixed Sugar as Fermentation Feedstock, with Use of Separation Membrane 2

Continuous fermentation was carried out using the *Candida utilis* CuLpLDH strain as the microorganism, with use of a separation membrane. The continuous fermentation was performed for 369 hours under the same conditions as in Example 1 except that the period of preculture was 40 hours and that the pH was adjusted to 6.0 with 1 N calcium hydroxide, to produce D-lactic acid (Table 5).

(Example 9) Production of D-Lactic Acid by Continuous Fermentation by *Candida utilis* Using Mixed Sugar as Fermentation Feedstock, with Use of Separation Membrane 3

Continuous fermentation was carried out using the *Candida utilis* CuLpLDH strain as the microorganism, with use of a separation membrane. The continuous fermentation was performed for 370 hours under the same conditions as in Example 1 except that the period of preculture was 30 hours and that the pH was adjusted to 6.0 with 1 N calcium hydroxide, to produce D-lactic acid (Table 5).

[Table 5]

| | Comparative Example 7: batch fermentation | Comparative Example 8: batch fermentation | Comparative Example 9: continuous fermentation | Example 7: continuous fermentation using membrane | Example 8: continuous fermentation using membrane | Example 9: continuous fermentation using membrane |
|---|---|---|---|---|---|---|
| Fermentation period (hr) | 40 | 40 | 368 | 370 | 369 | 370 |
| Total glucose fed (g) | 107 | 46.5 | 559 | 575 | 614 | 583 |
| Total xylose fed (g) | 0 | 62.1 | 773 | 781 | 795 | 753 |
| Unused glucose (g) | 0 | 0 | 0 | 0 | 0 | 0 |
| Unused xylose (g) | 0 | 0 | 83 | 93 | 61 | 15 |

(continued)

|  | Comparative Example 7: batch fermentation | Comparative Example 8: batch fermentation | Comparative Example 9: continuous fermentation | Example 7: continuous fermentation using membrane | Example 8: continuous fermentation using membrane | Example 9: continuous fermentation using membrane |
|---|---|---|---|---|---|---|
| Total production of D-lactic acid (g) | 30.9 | 22.8 | 287 | 353 | 391 | 344 |
| D-lactic acid yield (g/g) | 0.29 | 0.21 | 0.23 | 0.28 | 0.29 | 0.26 |

(Comparative Example 10) Production of Ethanol by Batch Fermentation by *Candida tropicalis* Using Mixed Sugar as Fermentation Feedstock 2

Batch fermentation was carried out using the *Candida tropicalis* NBRC0199 strain. As the fermentation medium, YPDX medium having the same composition as shown in Table 2 except that the sugar concentrations were 1% glucose and 9% xylose was used. Ethanol was produced by 42 hours of batch fermentation in which other operating conditions and the like were the same as in Comparative Example 2 (Table 6).

(Comparative Example 11) Production of Ethanol by Continuous Fermentation by *Candida tropicalis* Using Mixed Sugar as Fermentation Feedstock 2

Using the *Candida tropicalis* NBRC0199 strain, continuous fermentation was carried out without use of a separation membrane. As the fermentation medium, YPDX medium having the same composition as shown in Table 2 except that the sugar concentrations were 1% glucose and 9% xylose was used. Ethanol was produced by 400 hours of continuous fermentation in which other operating conditions and the like were the same as in Comparative Example 3 (Table 6).

(Example 10) Production of Ethanol by Continuous Fermentation by *Candida tropicalis* Using Mixed Sugar as Fermentation Feedstock, with Use of Separation Membrane 4

Using the *Candida tropicalis* NBRC0199 strain, continuous fermentation was carried out with use of a separation membrane. As the fermentation medium, YPDX medium having the same composition as shown in Table 2 except that the sugar concentrations were 1% glucose and 9% xylose was used. Ethanol was produced by 420 hours of continuous fermentation in which other operating conditions and the like were the same as in Example 1 (Table 6).

(Example 11) Production of Ethanol by Continuous Fermentation by *Candida tropicalis* Using Mixed Sugar as Fermentation Feedstock, with Use of Ceramic Separation Membrane 5

Using the *Candida tropicalis* NBRC0199 strain, continuous fermentation was carried out with use of a ceramic separation membrane. As the fermentation medium, YPDX medium having the same composition as shown in Table 2 except that the sugar concentrations were 1% glucose and 9% xylose was used. The *Candida tropicalis* NBRC0199 strain was cultured in 2 mL of YPD medium in a test tube at 30°C overnight with shaking (pre-pre-preculture). The obtained culture liquid was inoculated to 50 mL of YPD medium placed in a 500-mL baffled Erlenmeyer flask, and culture was performed overnight with shaking (pre-preculture). The pre-preculture liquid was inoculated to 1.5 L of YPDX medium placed in a membrane-separation-type continuous fermentation apparatus (the apparatus shown in Fig. 12 of WO2012/086763), and culture was performed for 36 hours with stirring at 800 rpm by the stirrer attached to the culture vessel while the aeration rate and the temperature in the culture vessel were controlled (preculture). Immediately after completion of the preculture, continuous fermentation was started. While the transmembrane pressure difference during filtration was kept at not more than 500 kPa, ethanol was produced by 400 hours of continuous fermentation (Table 6).

Culture vessel capacity: 2 L

Effective capacity of the culture vessel: 1.5 L

Separation membrane used: Celfit microfiltration membrane Monolith φ4-19 (NGK Insulators, Ltd.)

Length of the membrane separation element: 500 mm

Average pore size of the separation membrane: 0.1 μm

Temperature adjustment: 30 °C

Aeration rate in the culture vessel: 0.01 wm, compressed air

Stirring rate in the culture vessel: 800 rpm

pH Adjustment: none

[Table 6]

|  | Comparative Example 10: batch fermentation | Comparative Example 11: continuous fermentation | Example 10: continuous fermentation using membrane | Example 11: continuous fermentation using ceramic membrane |
|---|---|---|---|---|
| Fermentation period (hr) | 42 | 400 | 420 | 400 |
| Total glucose fed (g) | 16.5 | 210 | 227 | 41.2 |
| Total xylose fed (g) | 137 | 1802 | 1901 | 362 |
| Unused glucose (g) | 0 | 0 | 0 | 0 |
| Unused xylose (g) | 0 | 119 | 45.2 | 17.1 |
| Total production of ethanol (g) | 7.4 | 116 | 260 | 145 |
| Ethanol yield (g/g) | 0.05 | 0.06 | 0.13 | 0.12 |

(Comparative Example 12) Production of Ethanol by Batch Culture of *Candida tropicalis* Using Mixed Sugar as Fermentation Feedstock 3

Batch fermentation was carried out using the *Candida tropicalis* NBRC0199 strain. As the fermentation feedstock, a cellulose saccharification liquid prepared by the preparation method disclosed in Example 2 of WO2010/067785 using a nanofiltration membrane was used. The composition of the fermentation medium was adjusted as shown in Table 7 using reagents as appropriate. Ethanol was produced by 72 hours of batch fermentation in which other operating conditions and the like were the same as in Comparative Example 3 (Table 8).

[Table 7]

| Components | Content |
|---|---|
| Glucose | 6% |
| Xylose | 3% |
| Bactopeptone | 2% |

(continued)

| Components | Content |
|---|---|
| Yeast extract | 1% |

(Comparative Example 13) Production of Ethanol by Continuous Fermentation by *Candida tropicalis* Using Mixed Sugar as Fermentation Feedstock 3

Using the *Candida tropicalis* NBRC0199 strain, continuous fermentation was carried out without use of a separation membrane. Similarly to Comparative Example 12, the culture medium described in Table 7 was used as the fermentation medium. Ethanol was produced by 400 hours of continuous fermentation in which other operating conditions and the like were the same as in Comparative Example 3 (Table 8).

(Example 12) Production of Ethanol by Continuous Fermentation by *Candida tropicalis* Using Mixed Sugar as Fermentation Feedstock, with Use of Separation Membrane 6

Using the *Candida tropicalis* NBRC0199 strain, continuous fermentation was carried out with use of a separation membrane. Similarly to Comparative Example 12, the culture medium described in Table 7 was used as the fermentation medium. Ethanol was produced by 456 hours of continuous fermentation in which other operating conditions and the like were the same as in Example 1 (Table 8).

[Table 8]

| | Comparative Example 12: batch fermentation | Comparative Example 13: continuous fermentation | Example 12: continuous fermentation using membrane |
|---|---|---|---|
| Fermentation period (hr) | 72 | 400 | 456 |
| Total glucose fed (g) | 91.5 | 1220 | 1391 |
| Total xylose fed (g) | 43.5 | 580 | 661 |
| Unused glucose (g) | 0 | 0 | 0 |
| Unused xylose (g) | 0 | 58.0 | 29.0 |
| Total production of ethanol (g) | 37.6 | 525 | 699 |
| Ethanol yield (g/g) | 0.28 | 0.30 | 0.35 |

As shown by these results, chemical products could be produced with high yield from a mixed sugar of pentose and hexose.

INDUSTRIAL APPLICABILITY

By the present invention, the efficiencies of fermentation production of various chemical products using a fermentation feedstock containing pentose and hexose can be largely increased.

**Claims**

1. A method for producing a chemical product by continuous fermentation, said method comprising filtering a culture liquid of a microorganism(s) through a separation membrane; retaining unfiltered liquid in, or refluxing unfiltered liquid to, the culture liquid; adding a fermentation feedstock to the culture liquid; and recovering a product in the filtrate, wherein said fermentation feedstock contains pentose and hexose, and wherein said microorganism(s) is/are a microorganism(s) having a pathway in which pentose reductase and pentol dehydrogenase are used to metabolize pentose.

2. The method for producing a chemical product according to claim 1, comprising performing continuous fermentation under conditions where the oxygen transfer coefficient (KLa) is 5 to 300 h$^{-1}$.

3.  The method for producing a chemical product according to claim 1 or 2, wherein the ratio between the pentose and hexose contained in said fermentation feedstock is 1:9 to 9:1.

4.  The method for producing a chemical product according to claim 1 or 2, wherein said fermentation feedstock comprises a biomass-derived sugar liquid.

5.  The method for producing a chemical product according to any one of claims 1 to 4, wherein said pentose is xylose.

6.  The method for producing a chemical product according to any one of claims 1 to 5, wherein said pentose reductase is xylose reductase.

7.  The method for producing a chemical product according to any one of claims 1 to 6, wherein said pentol dehydrogenase is xylitol dehydrogenase.

## INTERNATIONAL SEARCH REPORT

| International application No. |
| --- |
| PCT/JP2013/050437 |

**A. CLASSIFICATION OF SUBJECT MATTER**
*C12P7/06*(2006.01)i, *C12P7/56*(2006.01)i

According to International Patent Classification (IPC) or to both national classification and IPC

**B. FIELDS SEARCHED**

Minimum documentation searched (classification system followed by classification symbols)
C12P7/06, C12P7/56

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)
CAPLUS/REGISTRY/MEDLINE/EMBASE/BIOSIS/WPIDS(STN),
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 4770987 B1 (Toray Industries, Inc.), 14 September 2011 (14.09.2011), paragraphs [0118], [0122], [0208]; example 1 (Family: none) | 1–7 |
| Y | WO 2007/097260 A1 (Toray Industries, Inc.), 30 August 2007 (30.08.2007), claims; paragraphs [0003] to [0012] & JP 2007-252367 A    & US 2009/0269812 A1 & EP 1988170 A1    & KR 10-2008-0096710 A & CN 101553572 A | 1–7 |
| Y | BICHO et al., Induction of Xylose Reductase and Xylitol Dehydrogenase Activities in Pachysolen tannophilus and Pichia stipitis on Mixed Sugars, Appl Environ Microbiol, Vol.54, No.1, 1988, pp.50-54 | 1–7 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 08 March, 2013 (08.03.13) | Date of mailing of the international search report 19 March, 2013 (19.03.13) |
| --- | --- |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

| International application No. |
|---|
| PCT/JP2013/050437 |

C (Continuation). DOCUMENTS CONSIDERED TO BE RELEVANT

| Category* | Citation of document, with indication, where appropriate, of the relevant passages | Relevant to claim No. |
|---|---|---|
| A | KARHUMAA et al., Comparison of the xylose reductase-xylitol dehydrogenase and the xylose isomerase pathways for xylose fermentation by recombinant Saccharomyces cerevisiae, Microbial Cell Factories, 2007, Vol.6,No.5, pp.1-10 | 1-7 |
| A | JP 2009-112289 A (National Institute of Advanced Industrial Science and Technology), 28 May 2009 (28.05.2009), claims (Family: none) | 1-7 |
| A | JP 2009-28030 A (Gekkeikan Sake, Co., Ltd.), 12 February 2009 (12.02.2009), paragraph [0013] (Family: none) | 2-7 |
| A | JP 2011-139707 A (Nippon Suisan Kaisha, Ltd.), 21 July 2011 (21.07.2011), paragraph [0025] & US 2005/0287651 A1    & EP 1549753 A & WO 2004/033698 A2    & CA 2501880 A & KR 10-2005-0055761 A | 2-7 |
| A | KIM et al., Batch and continuous fermentation of succinic acid from wood hydrolysate by Mannheimia succiniciproducens MBEL55E, Enzyme and Microbial Technology, 2004, Vol.35, pp.648-653 | 1-7 |
| A | FOND et al., The acetone butanol fermentation on glucose and xylose. I. Regulation and kinetics in batch cultures, Biotechnology and Bioengineering, 1986, Vol.XXVIII, pp.160-166 | 1-7 |

REFERENCES CITED IN THE DESCRIPTION

**Patent documents cited in the description**

- WO 2010067785 A
- JP HI1506934 A
- JP 2005229821 A
- JP 2003212888 A
- JP 2001095597 A
- JP 3041380 B
- JP 2009112289 A
- WO 2007097260 A
- WO 2012086763 A
- WO 2010140602 A

**Non-patent literature cited in the description**

- **SUSAN T. TOON et al.** Enhanced Cofermentation of Glucose and Xylose by Recombinant Saccharomyces Yeast Strains in Batch and Continuous Operating Modes. *Applied Biochemistry and Biotechnology.,* 1997, vol. 63-65, 243-255
- **A. ADEN et al.** Lignocellulosic Biomass to Ethanol Process Design and Economics Utilizing Co-Current Dilute Acid Prehydrolysis and Enzymatic Hydrolysis for Corn Stover. *NREL Technical Report,* 2002
- **GREGORY N. STEPHANOPOULO et al.** Metabolic Engineering: Principles and Methodologies. Tokyo Denki University Press, 2002, 174
- Laboratory Manual for Bioengineering. The Society for Biotechnology. Baifukan Co., Ltd, 1992, 310